# EUROPÄISCHE PATENTSCHRIFT

(11) **EP 0 876 297 B1**
(45) Veröffentlichungstag und Bekanntmachung des Hinweises auf die Patenterteilung: **12.12.2001**
(21) Anmeldenummer: 96945814.0
(22) Anmeldetag: 16.10.1996
(51) Int. Cl.: C02F 1/26, B09C 1/02, B01F 17/00

(54) **VERFAHREN ZUR RÜCKGEWINNUNG GEREINIGTER TENSIDE AUS WASCHLÖSUNGEN, INSBESONDERE ZUR WÄSCHE KONTAMINIERTEN ERDBODENS**
PROCESS FOR RECOVERING PURIFIED IONIC SURFACTANTS FROM WASHING SOLUTIONS, IN PARTICULAR FOR WASHING CONTAMINATED SOIL
PROCEDE DE RECUPERATION D'AGENTS TENSIOACTIFS IONIQUES PURIFIES CONTENUS DANS DES SOLUTIONS DE LAVAGE, NOTAMMENT POUR LAVER DES SOLS CONTAMINES

(30) Priorität: 17.10.1995 DE 19538592
(43) Veröffentlichungstag der Anmeldung: 11.11.1998
(73) Patentinhaber: FORSCHUNGSZENTRUM JÜLICH GMBH, 52425 Jülich (DE); Universität Bayreuth, 95447 Bayreuth (DE); Gallasch, Bernd, 95448 Bayreuth (DE)
(72) Erfinder: GALLASCH, Bernd, D-95448 Bayreuth (DE); NOMAYO, Matthias, D-95445 Bayreuth (DE); WOKAUN, Alexander, CH-5304 Endingen (CH); HAEGEL, Franz-Hubert, D-52349 Düren (DE)
(86) Internationale Anmeldenummer: DE9601985
(87) Internationale Veröffentlichungsnummer: WO9714653

(56) Entgegenhaltungen:
- WO-A-93/05013
- DE-A- 1 794 010
- DE-C- 4 323 146
- US-A- 3 734 776
- US-A- 5 427 688
- US-A- 5 429 773
- ACS SYMPOSIUM SERIES, Bd. 594, 13.März 1994, WASHINGTON, Seiten 231-248, XP000675758 YIN ET AL.: "Recovery of a Dialkyl Diphenyl Ether Disulfonate Surfactant from Surfactant Flush Solutions by Precipitation" in der Anmeldung erwähnt

## Beschreibung

Die Erfindung bezieht sich auf ein Verfahren zur Rückgewinnung gereinigter Tenside aus Waschlösungen, die mit organischen Schadstoffen belastete Tenside enthalten. Die Tenside werden aus den Waschlösungen durch Einstellen der Waschlösung auf einen Zustand unterhalb der Micellenbildungstemperatur (cmt) als dabei gebildeter Tensidniederschlag abgetrennt. Gegenstand der Erfindung ist auch eine Vorrichtung zur Durchführung des Verfahrens.

Waschlösungen der vorgenannten Art entstehen z.B. bei der Fahrzeugwäsche, bei Wäschen verschmutzter Industrieteile, bei Gebäudedekontamination oder insbesondere als Abwasser bei Wäschen kontaminierten Erdbodens. Die Tenside, die den Waschlösungen zugesetzt sind, extrahieren die organischen Schadstoffe, so daß diese in den Tensidlösungen solubilisiert zusammen mit dem Abwasser abgeführt werden können. Zur Konzentration der Schadstoffe und zugleich mit dem Ziel einer Regeneration der Waschlösung und ihrer Wiederverwendung werden die Waschlösungen aufgearbeitet.

Aus einer Veröffentlichung von A.N. Clarke et al., "Soil Clean-Up by Surfactant Washing", in Separation Science and Technology, 28 (13+14), S. 2103 ff., 1993, ist es bekannt, zur Reinigung von mit Biphenyl und Toluol verunreinigten Erdbodens oberflächenaktive Waschlösungen zu verwenden. Als Waschlösung wurde eine SDS (Sodium-Dodecyl-Sulfat)-Lösung eingesetzt, die nach Regeneration durch Strippen und Extraktion mit Heptan wiederverwendet werden konnte. Es handelt sich dabei um Flüssig-Flüssig-Extraktionsverfahren.

Die DE 43 231 46 C1 zeigt ein Verfahren zur Dekontaminierung von Feststoffen mittels Lösungsmittelextraktion, bei dem die mit schwer löslichen anorganischen Verbindungen kontaminierten Stoffe in einem Reaktor mit einem auf die jeweilige Kontamination abgestimmten Lösungsmittel in intensiven Kontakt gebracht werden und direkt und gezielt auf Temperaturen aufgeheizt werden, bei denen ein maximales Löslichkeitsprodukt der kontaminierten Stoffe vorliegt.

Die DE-OS 179 40 10 offenbart ein Verfahren zum Konzentrieren von Lösungen durch kontinuierliches Ausfrieren des Lösungsmittels, wobei sich die Lösungsmittelkristalle an einer gekühlten Fläche bilden, die durch Abschaben entfernt und im Gegenstrom zur ausfrierenden Lösung geführt werden, bei dem erfindungsgemäß die Gegenstromführung der Lösungsmittelkristalle unmittelbar nach deren Abschaben eingeleitet wird.

Zum Abtrennen der Tenside aus den Waschlösungen ist auch vorgeschlagen worden, die Micellenbildung der Tenside zu nutzen. Hierzu ist es erforderlich, die die Tenside gelöst enthaltenden Waschlösungen unter die Micellenbildungstemperatur (cmt) abzukühlen und die dabei ausgefällten Tensidmicellen aus den Waschlösungen abzufiltern. In einer Veröffentlichung von Y. Yin et al., "Recovery of a Dialkyl Diphenyl Ether Disulfonate Surfactant from Surfactant Flush Solutions by Precipitation", in ACS Symposium Series 594, San Diego, Californien, März 13/17, 1994, Am. Chem. Soc., 1995, Chapter 17, S. 231/248, ist angegeben, Waschlösungen mit bei Raumtemperatur löslichen Tensiden zur Tensidausfällung unter Raumtemperatur abzukühlen. Ein solches Abkühlen ist technisch und energetisch mit Aufwand verbunden, realisiert wird daher bisher die chemische Abtrennung der die Schadstofffracht enthaltenden Tenside.

Aufgabe der Erfindung ist es , ein Verfahren zur Regeneration von Waschlösungen, die mit Schadstoffen kontaminierte Tenside enthalten, zu schaffen, das ohne Probleme hinsichtlich der Bildung von Emulsionen eine wirtschaftlich effektive Verfahrensweise und ohne erhebliche Energieverluste eine weitgehend quantitative Trennung der Tenside erlaubt.

Diese Aufgabe wird bei einem Verfahren der eingangs genannten Art durch die im Patentanspruch 1 genannten Maßnahmen gelöst. Danach werden zum Ausfällen der Tenside unterhalb der kritischen Micellenbildungstemperatur (cmt) Tenside eingesetzt, deren cmt in der Waschlösung > 40°C beträgt. Dabei ist vorgesehen, den jeweils abgetrennten Tensidniederschlag mit Hilfe eines organischen Lösungsmittels zu extrahieren und dabei die im Tensid enthaltenden Schadstofe im organischen Lösungsmittel zu binden. Über dieser Micellenbildungstemperatur liegen die Tenside in der Waschlösung gelöst vor. Kühlt sich die Waschlösung auf Raumtemperatur ab, werden die Tenside ausgefällt und können abgefiltert werden. In Betracht kommen Tenside, deren Kopfgruppe anionische (z.B. Sulforyl-, Carboxyl-), kationische (z.B. Ammonium-), nicht ionische (z.B. Zucker-,Ethoxy-) oder amphotere (zwitterionische z.B. Betain-, Sulfobetain -) Gruppen enthält. Die Schwanzgruppe besteht aus organischem Rest, der Methylengruppen, Ethylengruppen und/oder aromatische Gruppen enthält. Bei anionischen, kationischen oder amphoteren Kopfgruppen sind die entsprechenden Ladungen und Gegenionen zu berücksichtigen. Technische Tenside können mono- und bifunktionell aufgebaut sein.

Wird zur Wäsche als Waschlösung eine wässrige alkoholische Tensidlösung verwendet, so läßt sich das Tensid mit den im Tensid gebundenen organischen Schadstoffen nach dem Waschprozeß durch Erniedrigen der Alkoholkonzentration in der Waschlösung ausfällen, wenn die Alkoholkonzentration derart gesenkt wird, daß die cmt des Tensids über die Prozeßtemperatur des Waschprozesses ansteigt (Patentanspruch 2). Mit Verringerung der Alkoholkonzentration bilden sich dann in der Waschlösung ohne Temperaturänderung Tensidmicellen, die abzufiltern sind.

Mit solchen wässrig alkoholischen Tensidlösungen sind Wäschen kontaminierten Erdbodens bei Bodentemperatur möglich. Eine Bodenwäsche kann gemäß dieser Verfahrensmaßnahmen somit "in-situ", d.h. vor Ort ohne Bodenaushub durchgeführt werden. Bisher gebräuchliche Zubereitungen für die Bodenwäsche sind mit dem Problem verbunden, daß Rückstände des Extraktionsmittels im Erdboden verbleiben und ebenfalls verbliebene Rückstände des Schadstoffs mobilisieren, so daß die Auswaschung von Schadstoffen aus dem behandelten Bodenmaterial ins Grundwasser unter Umständen höher sein kann als vor der Behandlung. Das Problem wird nach vorgenanntem Patentanspruch 2 durch Verwendung einer Wasser/Alkohol-Tensidlösung als Waschlösung dadurch behoben, daß nach der Behandlung des Bodenmaterials mit der Waschlösung und anschließender weitestgehenden Entfernung des belasteten Tensids durch Spülung mit dem Wasser/Alkohol-Gemisch im Wasser/Alkohol-Gemisch die Alkoholkonzentration durch Zugabe weiteren Wassers gesenkt wird, so daß das Tensid ausfällt und somit nicht mehr in der Lage ist, Schadstoffe aus dem Boden zu transportieren. Nach Abtrennen des Tensidniederschlags erfolgt eine Immobilisierung des noch verbliebenen Rückstands durch Spülung mit Klarwasser, so daß nach nahezu vollständiger Auswaschung des restlichen Alkohols im gereinigten Erdboden die kritische Micellenbildungstemperatur auf die cmt des verwendeten Tensids in wässriger Lösung, also cmt ≥ 40°C ansteigt. Eine Umweltbelastung durch noch verbliebenes restliches Tensid ist somit nicht gegeben.

Erfindungsgemäß ist es vorgesehen, den jeweils abgetrennten Tensidniederschlag mit Hilfe eines organischen Lösungsmittels zu extrahieren, und dabei die im Tensid enthaltenden Schadstoffe im organischen Lösungsmittel zu binden. Das Lösungsmittel kann durch Destillation zurückgewonnen werden, wobei die Schadstoffe aufkonzentriert werden. Als Extraktionsmittel sind hierzu beispielsweise Ether, Essigsäureethylester, Dichlormethan geeignet. Das extrahierte, von Schadstoffen gereinigte Tensid wird nach Patentanspruch 3 dem Waschwasser, das nach Abtrennung des ausgefällten Tensidniederschlags verbleibt, wieder zugeführt. Es entsteht so ein geschlossener Waschwasser-, Tensid- und Lösungsmittelkreislauf, aus dem die Schadstoffe entfernt werden.

Die Erfindung und weitere Ausgestaltungen der Erfindung sowie eine zum Erfindungsgegenstand gehörende Vorrichtung zur Durchführung des Verfahrens werden nachfolgend anhand von Ausführungsbeispielen näher erläutert. Die Zeichnung zeigt:

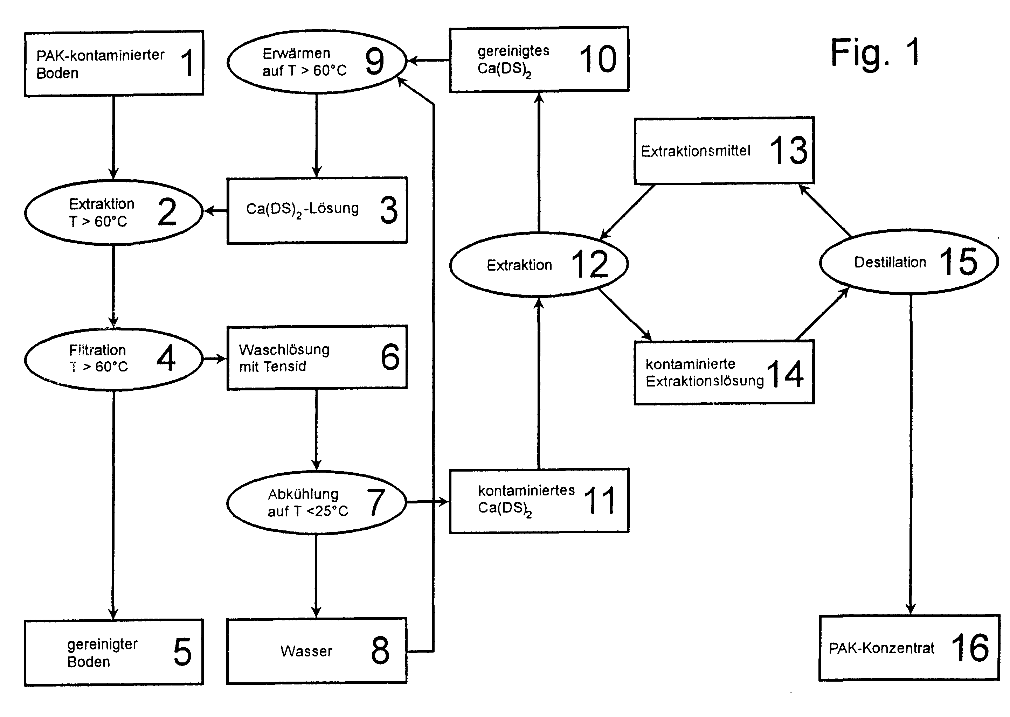

- Figur 1:: Fließschema einer Anlage zur Erdbodenreinigung mit geschlossenem Waschlösungs-, Tensid- und Lösungsmittelkreislauf;

In Figur 1 ist ein Fließschema einer Anlage zum Reinigen durch organische Schadstoffe verunreinigten, beispielsweise PAK(Polyzyklische Aromatische Kohlenwasserstoffe)-kontaminierten Erdbodens wiedergegeben. Als Extraktionsmittel zur Bindung der im Erdboden enthaltenen Schadstoffe wird als Tensid in der Waschlösung Calciumdodecylsulfat Ca(DS)₂ benutzt. Die Waschlösung wird in der Anlage im Kreislauf geführt. Ca(DS)₂ weist eine kritische Micellenbildungstemperatur cmt von 60°C auf, die Bodenreinigungsanlage nach Figur 1 arbeitet daher-bis auf das Ausfällen der Tenside durch Abkühlung der Waschlösung unter die cmt - bei einer Temperatur T > 60°C.

Der PAK-kontaminierte Boden wird bei der Anlage nach Figur 1 aus einem Erdbodenlager 1 in einen Mischer 2 gegeben, dem die Ca(DS)₂-Waschlösung zur Extraktion der Schadstoffe bei einer Temperatur T > 60°C aus einem Vorratsbehälter 3 zugeführt wird. Dem Mischer 2 folgt ein Filter 4, der die im Mischer 2 mit den Schadstoffen belastete Tensidlösung vom gereinigten Boden trennt. Der gereinigte Boden wird zu einer Erdbodenausgabe 5 transportiert, die Waschlösung mit kontaminiertem Tensid fließt in einen Zwischenbehälter 6 ab. Aus dem Zwischenbehälter wird die Waschlösung in einen Kühler 7 eingeführt und auf eine Temperatur T < 25°C abgekühlt, so daß das mit Schadstoffen belastete Tensid unterhalb der cmt als Feststoff ausfällt und ein von Schadstoffen weitgehend freier Waschlösungsrückstand, im Ausführungsbeispiel Wasser, aus dem Kühler 7 in einen Waschlösungsbehälter 8 abfließen kann. Vom Waschlösungsbehälter 8 strömt der Waschlösungsrückstand in einen Erhitzer 9, wo er auf eine Temperatur T > 60°C erwärmt wird. Im Erhitzer 9 wird der Waschlösungsrückstand wieder mit frischem, im Ausführungsbeispiel mit wiedergewonnenem bzw. ergänzten frischen Tensid aus einem Tensidbehälter 10 gemischt und im Kreislauf zurück in den Vorratsbehälter 3 gegeben, aus dem er als Waschlösung für den zu reinigenden Erdboden dann erneut eingesetzt werden kann.

Das als Feststoff im Kühler 7 ausgefällte, schadstoffbelastete Tensid wird abgetrennt und in einen Schadstoffzwischenbehälter 11 gegeben. Aus dem Schadstoffzwischenbehälter wird das Tensid in eine Extraktionseinheit 12 eingeführt, in die aus einem Extraktionsmittelvorratsbehälter 13 ein Extraktionsmittel zufließt. Mit dem Extraktionsmittel werden aus dem schadstoffbelasteten Tensid die Schadstoffe ausgetragen. Der schadstofffreie Tensidfeststoff wird in den Tensidbehälter 10 überführt, während das Extraktionsmittel über einen Lösungsmittelbehälter 14 in einen Destillator 15 abfließt und hier von den Schadstoffen getrennt wird. Die Schadstoffe werden in einem Schadstofflager 16 gesammelt und anschließend entsorgt, z.B. verbrannt. Das aus dem Destillator 15 gewonnene gereinigte Extraktionsmittel wird in den Extraktionsmittelvorratsbehälter 13 und aus ihm in die Extraktionseinheit 12 eingeleitet. Auch das Extraktionsmittel für das schadstoffbelastete Tensid wird also im Kreislauf geführt.

Die in Figur 1 gezeigte Anlage arbeitet somit bei vollständig geschlossenem Waschlösungs-, Tensid- und Lösungsmittelkreislauf. Das zur Reinigung des kontaminierten Erdbodens eingesetzte Tensid dient als Extraktionsmittel und Transportmedium für die Schadstoffe. Aus dem Tensid werden die Schadstoffe durch erneute Extraktion ausgetragen. Auch dieses Extraktionsmittel wird wiedergewonnen und im Kreislauf geführt. Die vom Tensid aus dem Boden extrahierten Schadstoffe können so in vorteilhafter Weise sehr konzentriert gewonnen werden.

In einer Laboranlage nach Figur 1 wurden im Mischer 2 30 g eines Feinkornanteils aus einer Bodenwaschanlage mit 500 ml einer 0,1 M Calciumdodecylsulfat-Lösung bei 80°C gerührt. Durch Filtration der heißen Suspension wurde zunächst das Bodenmaterial abgetrennt. Anschließend wurde durch Abkühlung Calciumdodecylsulfat Ca(DS)₂ ausgefällt und ebenfalls durch Filtration entfernt.

Die ursprüngliche Belastung des Feinkornanteils betrug entsprechend einer Heißextraktion mit Toluol: 96 mg/kg Naphthalin, 62 mg/kg Acenapththen, 129 mg/kg Fluoren, 575 mg/kg Phenanthren, 244 mg/kg Anthracen, 888 mg/kg Fluoranthen, 402 mg/kg Pyren, 276 mg/kg Benzo[b]fluoranthen, 155 mg/kg Benzo[k]fluoranthen, 260 mg/kg Benzo[a]pyren, 24 mg/kg Dibenzo[a,h]anthracen, 119 mg/kg Benzo [ghi] perylen und 168 mg/kg Indeno[1,2,3-cd]pyren. Dies sind in der Summe 4142 mg/kg. Davon befanden sich nach der Abtrennung von Ca(DS)₂ noch zwischen 0 und 76 % in der wäßrigen Lösung, wobei der höchste Wert für Pyren und die niedrigsten Werte für Naphthalin und Benzo[ghi]perylen gefunden wurden.

Die Konzentrationen im Wasser, das nach Abtrennen des Tensids im Kühler 7 in den Waschlösungsbehälter 8 eingeführt wurde, konnten im Ausführungsbeispiel durch vorausgehendes Nachfällen aus der vom Kühler 7 ablaufenden Waschlösung mit 1 ml gesättigter Calciumacetatlösung auf 60 ml Lösung von maximal 23 mg/l auf unter 0,3 mg/l PAK vermindert werden. Durch zusätzliche Extraktion der wäßrigen Phase mit Essigester konnten die PAK-Gehalte unter die Nachweisgrenze gedrückt werden. In Figur 1 ist diese Verfahrensvariante nicht wiedergegeben.

Das im Kühler 7 gefällte Ca(DS)₂ (ca. 17 g, d.h. 62 % der eingesetzten Menge) wurde in der Extraktionseinheit 12 mit 500 ml Methylenchlorid (= Dichlormethan) extrahiert. Die PAK gingen dabei quantitativ in das organische Extraktionsmittel über. Der extrahierte Anteil betrug jedoch für alle PAK unter 1 % der Menge am eingesetzten Bodenmaterial.

Ein Teil des Ca(DS)₂ war bereits bei der Heißfiltration des Bodens ausgefallen, da mit den benutzten Laborgeräten eine Abkühlung während der Filtration nicht gewährleistet werden konnte. Das Bodenmaterial wurde deshalb vor der Analyse viermal mit 500 ml Wasser ausgewaschen, getrocknet und auf den PAK-Gehalt untersucht. Eine Bilanzierung der Massen in der Waschlösung vor der Nachfällung und im extrahierten Boden ergab Ausbeuten zwischen 58 % für Anthracen und 148 % für Indeno[1,2,3-cd]pyren. Ausbeuten über 100 % erklären sich durch unvollständige Extraktionen mit Toluol, deren Werte als Bezugspunkt gewählt wurden.

In einem zweiten Extraktionsschritt wurden 10 g des Bodenmaterials mit 300 ml 0,1 M Ca(DS)₂-Lösung extrahiert. Die Restgehalte im Boden lagen nach diesem Schritt zwischen 0 % für Acenaphthen und 60 % für Indeno[1,2,3-cd]pyren. Mit Ausnahme von Naphthalin, das noch in nennenswerten Gehalten auftrat, ist die Extraktion für die niederen PAK (3 und vier Ringe) am besten. Im einzelnen ergaben sich folgende Gehalte: 50 mg/kg Naphthalin, Acenaphthen nicht nachweisbar, 9 mg/kg Fluoren, 37 mg/kg Phenanthren, 18 mg/kg Anthracen, 111 mg/kg Fluoranthen, 73 mg/kg Pyren, 83 mg/kg Benzo[a]anthracen, 83 mg/kg Chrysen, 124 mg/kg Benzo[b]fluoranthen, 52 mg/kg Benzo[k]fluoranthen, 101 mg/kg Benzo[a]pyren, Dibenzo[a,h]anthracen nicht nachweisbar, 66 mg/kg Benzo[ghi]perylen und 101 mg/kg Indeno[1,2,3-cd]pyren, in der Summe 908 mg/kg.

Als allgemeine chemische Formel für die für das erfindungsgemäße Verfahren in Frage kommenden Tenside läßt sich angeben:
Für monofunktionelle Tenside
   H-A_{a,Δ}-B_{b}-C-D_{d}-E,
für bifunktionelle Tenside
   E-D_{d}-C-B_{b}-A_{aΔ}-Sp-A_{a,Δ}-B_{b}-C-D_{d}-E,
wobei die Großbuchstaben die molekularen Grundbausteine, die tiefgestellten Indizes die Anzahl der Grundbausteine oder mit Δ die Zahl der Doppelbindungen im Molekül symbolisieren. Die hydrophoben Molekülbestandteile sind:
H = Wasserstoffatom;
Sp = Sauerstoff (O) oder Schwefelatom (S) als beweglicher Spacer;
A_{a,Δ} = aus Methylengruppen-(CH2)- aufgebauter Alkylrest, der 0 bis 4 Doppelbindungen enthalten kann. Allgemeine Summenformel (n=Atomanzahl 6 bis 20) :
   Δ=0 CₙH₂ₙ
   Δ=1 CₙH₂ₙ₋₂
   Δ=2 CₙH₂ₙ₋₄
   Δ=3 CₙH₂ₙ₋₆
   Δ=4 CₙH₂ₙ₋₈

### B_{b}

Arylrest, der aus unterschiedlich (z.B. 1,2 oder 1,4) mit der Alkylkette verknüpften Phenyl- oder Naphthylgruppen mit b=0, 1 oder 2 besteht.

### C

Zur Kopfgruppe überleitender Alkylamino, Acyl- oder Acylaminorest, der aus einem kurzen Alkylrest (wie bei Aₐ mit n=0 bis 5) und einer polaren Gruppe besteht. Diese Bausteine treten pro Molekül nur einmal auf. Die polare Gruppe ist mit der hydrophilen Kopfgruppe (meist mit Ethoxygruppen) verknüpft. Die polaren Bestandteile sind, eine
- NH-Gruppe bei Alkylamino-,
- CO-Gruppe bei Acyl- und
- CO-NH-Gruppe bei Acylaminoresten.

### D_{d}

Polare, nichtionische Gruppe mit d= 0 bis 6. Als Gruppen sind hier Zucker, Ethoxy- und Aminoxidgruppen möglich.
Bei den Zuckern sind dies (meist 1,4-verknüpfte) Mono-oder Oligozucker (z.B. aus Saccharose, Maltose, Fructose, Sorbit, Anhydrosorbit). Auch Zuckerphosphate, Zukkersulphate und Zuckeracetate kommen in technischen Tensiden zur Anwendung.
Bei Ethoxylaten sind (CH₂-CH₂-O)-Einheiten linear verknüpft.
Bei Aminoxiden ist eine (d=1)Aminodiethyl- (-Net₂-) oder Aminodimethylgruppe (-Nme₂-) als Donorgruppe mit einem Sauerstoffatom verknüpft.

### E

Pro Molekül eine polare anionische, kationische oder amphotere (zwitterionische) Gruppe.
Für anionische Tenside werden technisch
- Carboxyl: -COO⁻
- Sulfuryl-: -SO₃⁻
- Sulfat-: -O-SO₃⁻ oder
- Phosphatgruppen: -O-PO₃²⁻ eingesetzt.

Um die Eigenschaft "wasserlöslich bei Temperaturen oberhalb 40°C" einzustellen, können Tenside entsprechend der Polaritätsbeiträge der Bestandteile von Kopf-und Schwanzgruppen konfektioniert werden. Eine Verstärkung der hydrophoben Eigenschaften (entspricht eine Anhebung der cmt) kann z.B. durch Kettenverlängerung oder durch Abschwächung der Polarität der Kopfgruppe erreicht werden. Entsprechend kann eine Verstärkung der hydrophilen Eigenschaften (eine Absenkung der cmt) z.B. durch Einführung einer polareren Kopfgruppe erreicht werden. Durch Kombination der verschiedenen Baugruppen mit ihren Einzelbeiträgen zur Löslichkeit kann eine bestimmte cmt eingestellt werden. Bei technischen Produkten ist zu beachten, daß mit der Zunahme der verschiedenen Baugruppen mit mehr als einer Untereinheit vermehrt uneinheitliche Gemische durch unvollständigen Umsatz entstehen, die keine scharf definierte cmt mehr besitzen. Bei nichtionischen Tensiden wird die cmt durch das Tensid selbst bestimmt, bei ionischen Tensiden tragen dagegen die Gegenionen einen erheblichen Anteil an der resultierenden cmt. So verschieben Calcium-gegenionen die cmt des SDS ohne Änderung des eigentlichen Tensids auf ca. 60°C.

Als ein weiteres Ausführungsbeispiel zur Wäsche kontaminierten Erdbodens wird als Waschlösung eine wässrige alkoholische Tensidlösung eingesetzt. In 500 ml H₂O:CH₃CH₂OH=1:1 Wasser/Ethanol-Lösung werden O,1 M Calciumdodecylsulfat Ca(DS)₂ gelöst. Mit dieser Waschlösung wird der Erdboden gereinigt. Die nach dem Waschvorgang verbleibende Waschlösung, die die Tenside mit den extrahierten Schadstoffen gelöst enthält, wird abgezogen und zum Ausfällen der Tenside und zum Senken der Alkoholkonzentration in der Waschlösung mit Wasser derart verdünnt, daß sich ein Verhältnis H₂O:CH₃CH₂OH= 10 : 1 einstellt. Bei dieser Alkoholkonzentration steigt die cmt in der Waschlösung über cmt ≥ 40°C an, so daß sich in der Waschlösung Tensidmicellen bilden, die abgefiltert werden können. Zur Wiederverwendung des Tensids werden die Schadstoffe - in gleicher Weise wie bereits im Ausführungsbeispiel nach Figur 1 beschrieben - mit einem Extraktionsmittel extrahiert.

Die Alkoholkonzentration in der abgezogenen Waschlösung läßt sich statt durch Wasserzugabe auch durch Erwärmen der Waschlösung und Austreiben des Ethanols oder durch Druckreduzierung erniedrigen. Voraussetzung für dieses Vorgehen ist die Stabilität der Tenside bei dieser Prozedur.

Statt einer Waschlösung, die zur Erdbodenwäsche benutzt wurde, lassen sich in einer Anlage nach Figur 1 in gleicher Weise auch Waschlösungen aufbereiten, die zur Fahrzeugwäsche oder Wäsche verschmutzter Industrieteile oder dekontaminierter Gebäude eingesetzt wurden. Die Verfahrensparameter, insbesondere die Temperaturen für die Wäsche und Tensidausfällung, sind entsprechend dem jeweils verwendeten Tensid bzw. Tensidgemisch anzupassen. Als Tensid ist beispielsweise 1-Chlor-3-Hexanol, als kationisches Tensid 1-Amino-octa decan-Hydrochlorid einsetzbar, die beide oberhalb 40°C in Wasser gelöst sind.

### Bezugszeichenliste

- Erdbodenlager: 1
- Mischer: 2
- Vorratsbehälter: 3
- Filter: 4
- Erdbodenausgabe: 5
- Zwischenbehälter: 6
- Kühler: 7
- Waschlösungsbehälter: 8
- Erhitzer: 9
- Tensidbehälter: 10
- Schadstoffzwischenbehälter: 11
- Extraktionseinheit: 12
- Extraktionsmittelvorratsbehälter: 13
- Lösungsmittelbehälter: 14
- Destillator: 15
- Schadstofflager: 16

## Patentansprüche

1. Verfahren zur Rückgewinnung von Tensiden aus Waschlösungen, die mit organischen Schadstoffen belastete Tenside enthalten, wobei die Tenside aus der Waschlösung bei einer Temperatur unterhalb der kritischen Micellenbildungstemperatur der Tenside als dabei gebildeter fester Tensidniederschlag abgetrennt werden,
**dadurch gekennzeichnet,**
**daß** die behandelte waschlösung Tenside mit einer Micellenbildungstemperatur (cmt) in der Waschlösung von cmt ≥ 40°C beinhaltet, und daß der feste Tensidniederschlag mit Hilfe eines organischen Lösungsmittels extrahiert wird.

2. Verfahren nach Anspruch 1,
**dadurch gekennzeichnet,**
**daß** die Waschlösung aus einem waschprozess stammt, in dem eine wässrige alkoholische Tensidlösung verwendet wird, deren cmt nach dem Waschvorgang zum Ausfällen des Tensids durch Absenken des Alkoholgehaltes der Waschlösung über die Prozeßtemperatur erhöht wird.

3. Verfahren nach Anspruch 1 ,
**dadurch gekennzeichnet,**
**daß** das nach Abtrennung des Tensidniederschlags verbleibende Waschwasser mit von Schadstoffen gereinigtem, extrahierten Tensid vermischt und bei einer Temperatur oberhalb der Micellenbildungstemperatur als Waschlösung in einem waschprozeß wiederverwendet wird.

4. Verfahren nach Anspruch 2
**dadurch gekennzeichnet,**
**daß** die waschlösung aus einer Wäsche kontaminierten Erdbodens stammt und anionische Tenside mit mehrwertigen Kationen als Gegenionen in einer Wasser/Alkohollösung enthält.

## Claims

1. Process for recovering surfactants from washing solutions containing surfactants burdened by organic pollutants, the surfactants being removed from the washing solution, at a temperature below the critical micelle formation temperature of the surfactants, as a solid surfactant precipitate formed in the process,
**characterised in that** the treated washing solution contains surfactants with a micelle formation temperature (cmt) in the washing solution of cmt ≥ 40°C, and that the solid surfactant precipitate is extracted with the help of an organic solvent.

2. Process according to claim 1,
**characterised in that** the washing solution originates from a washing process in which an aqueous alcoholic surfactant solution is used whose cmt is increased after the washing operation in order to precipitate the surfactant by lowering the alcohol content of the washing solution via the process temperature.

3. Process according to claim 1,
**characterised in that** the washing water that is left following the removal of the surfactant precipitate is mixed with extracted tenside that has been purified of pollutants and is re-cycled as a washing solution in a washing process at a temperature above the micelle formation temperature.

4. Process according to claim 2,
**characterised in that** the washing solution originates from contaminated soil washing and contains anionic surfactants with multivalent cations as counterions in a water/alcohol solution.

## Revendications

1. Procédé de récupération d'agents tensioactifs dans des solutions de lavage, qui contiennent des agents tensioactifs chargés de substances organiques polluantes, les agents tensioactifs étant séparés de la solution de lavage, à une température inférieure à la température critique de formation de micelles des agents tensioactifs, sous la forme de précipités d'agents tensioactifs solides qui se forment,
**caractérisé en ce que** la solution de lavage traitée contient des agents tensioactifs ayant une température de formation de micelles (tfm) dans la solution de lavage de tfm ≥ 40°C, et **en ce que** le précipité solide d'agent tensioactif est extrait à l'aide d'un solvant organique.

2. Procédé suivant la revendication 1, **caractérisé en ce que** la solution de lavage provient d'une opération de lavage, dans laquelle une solution aqueuse alcoolique d'agent tensioactif à été utilisée, dont la tfm après l'opération de lavage est élevée au dessus de la température du processus, après l'opération de lavage pour la précipitation de l'agent tensioactif, par abaissement de la teneur en alcool de la solution de lavage.

3. Procédé suivant la revendication 1, **caractérisé en ce qu'**après la séparation du précipité d'agent tensioactif, l'eau de lavage restante est mélangée à de l'agent tensioactif extrait purifié des substances polluantes, et est réutilisée dans les opérations de lavage en tant que solution de lavage à une température supérieure à la température de formation de micelles.

4. Procédé suivant la revendication 2, **caractérisé en ce que** la solution de lavage provient d'un lavage de sol contaminé et contient des agents tensioactifs anioniques à cations plurivalents comme contre-ions dans une solution eau/alcool.
